# EUROPÄISCHE PATENTSCHRIFT

(11) **EP 1 541 106 B1**
(45) Veröffentlichungstag und Bekanntmachung des Hinweises auf die Patenterteilung: **16.04.2014**
(21) Anmeldenummer: 04028234.5
(22) Anmeldetag: 29.11.2004
(51) Int. Cl.: A61F 13/56, D04H 13/00

(54) **Verbundstoff für einen elastischen Windelverschluss und Vefahren zu seiner Herstellung**
composite material for an elastic fastener for diaper and production method of the same
matériau composite pour une fermeture élastique pour couche culotte et son procédé de production

(30) Priorität: 13.12.2003 DE 10358409
(43) Veröffentlichungstag der Anmeldung: 15.06.2005
(73) Patentinhaber: Mondi Gronau GmbH, 48599 Gronau (DE)
(72) Erfinder: Baldauf, Georg, 48366 Laer (DE)
(74) Vertreter: Albrecht, Rainer Harald

(56) Entgegenhaltungen:
- US-A- 4 879 170
- DATABASE WPI Week 199805 Thomson Scientific, London, GB; AN 1998-045823 XP002522741 & JP 09 294772 A (KAO CORP) 18. November 1997 (1997-11-18)

## Beschreibung

Die Erfindung betrifft einen Verbundstoff, insbesondere für elastische Windelverschlüsse.

Windeln besitzen oft mechanische Klettverschlüsse, die sich leicht verschließen und wieder öffnen lassen. Ein Teil des Klettverschlusses ist mit weiblichen Verschlusselementen versehen und im vorderen Bündchenbereich der Windel aufgeklebt. Ein zugehöriges Hakenband mit männlichen Verschlusselementen ist an Verschlusslaschen angebracht, die jeweils rechts und links am hinteren Bündchenbereich der Windel befestigt sind und in der Regel aus einem elastischen Material bestehen. Beim Schließen des Windelverschlusses und beim Tragen der Windel werden die elastischen Verschlusslaschen bisweilen überdehnt. Die Überdehnung ist mit einer bleibenden Dehnung des elastischen Materials verbunden und wirkt sich nachteilig auf die Passform der Windel aus.

Aus EP 0 809 992 B1 ist ein elastisches Verschlussband für Windelverschlüsse bekannt, das einen plastisch verformbaren Bereich in Form einer feuchtigkeitsdurchlässigen Folie und mehrere dehnbare Bereiche aufweist. Die dehnbaren Bereiche wechseln in Richtung der Breite des Bandes mit den plastisch verformbaren Bereichen ab und sind in Form einer Linie, eines Bandes oder einer Spirale bereitgestellt.

In DE 102 12 842 A1 ist ein Vliesmaterial mit elastischen Eigenschaften beschrieben, welches Fasern aus einem elastischen Polymer und Faseranteile aus einem nicht elastischen thermoplastischen Polymer enthält. Der elastische Vliesstoff kann unter anderem als Taillenband für Windeln verwendet werden. Windelverschlüsse werden beim Gebrauch häufig um mehr als das doppelte ihrer Ursprungslänge gedehnt und sind einer wesentlich größeren Dehnung unterworfen als das Taillenband einer Windel. Für Anwendungen, bei denen eine sehr große Dehnung gefordert ist, erscheint das Vliesmaterial als solches daher nicht geeignet.

Aus JP 09-294772 A ist ein Verbundstoff für elastische Abschnitte an einer Windel bekannt, der aus einer elastischen Trägerfolie mit einer bevorzugten Dehnungsrichtung und mindestens einer aufkaschierten Deckschicht aus einem Spinnvlies besteht. Die Filamente des Spinnvlieses weisen einen Filamentkern aus einem thermoplastischen Elastomer und einen Filamentmantel aus einem nichtelastischen thermoplastischen Polymer auf. Auch bei diesem Material besteht die Gefahr, dass ein aus dem Verbundstoff gefertigter Windelverschluss beim Schließen einer Windel überdehnt wird.

Vor dem eingangs beschriebenen Hintergrund besteht die der Erfindung zugrunde liegende Aufgabe darin, einen für elastische Windelverschlüsse geeigneten elastischen Verbundstoff anzugeben, bei dem eine Überdehnung durch unsachgemäßigen Gebrauch oder durch Beanspruchungen während eines bestimmungsgemäßen Gebrauchs weitgehend ausgeschlossen ist.

Erfindungsgemäß wird die Aufgabe gelöst durch einen Verbundstoff mit dem im Patentanspruch 1 angegebenen Merkmal. Der Verbundstoff besteht aus einer elastischen Trägerfolie, die eine bevorzugte Dehnungsrichtung aufweist, und mindestens einer aufkaschierten Deckschicht aus einem Faservlies, welches Fasern aus einem elastischen Polymer und durch eine Dehnung des Faservlieses in der bevorzugten Dehnungsrichtung verstreckte Faseranteile aus einem nichtelastischen thermoplastischen Polymer enthält.

Der erfindungsgemäße Verbundstoff zeichnet sich durch eine deutlich wahrnehmbare Dehngrenze aus. Der Verbundstoff kann bis an diese Grenze mit geringer Kraft gedehnt werden. Im Dehnungsbereich bis zu dieser Grenze stellt sich der Verbundstoff bei Entlastung elastisch zurück. Bei der Benutzung des Verbundstoffes als Windelverschlusslasche wird dieser Bereich während des Verschließens der Windel als elastischer Arbeitsbereich wahrgenommen. Bei Erreichen der durch das Material festgelegten Dehngrenze ist ein starker Anstieg der zur weiteren Verdehnung nötigen Kraft zu bemerken und der Windelverschluss verhält sich oberhalb der definierten Dehngrenze unelastisch. Dies wirkt sich vorteilhaft auf die Passform der Windel aus. Durch die Elastizität der Windelverschlusslasche kann der Windelverschluss den Bewegungen der Person entsprechend gedehnt werden und sich wieder entspannen. Dabei wird der Windelverschluss aufgrund der vorliegenden Dehngrenze nicht überdehnt, so dass die Passform der Windel während des Gebrauchs erhalten bleibt. Männliche Verschlusselemente können auf dem als Windelverschlusslasche zugeschnittenen Verbundstoff in Form eines Hakenbandes mittels Verkleben oder Verschweißen, z. B. durch Ultraschall, befestigt werden.

Die Elastizität des Verbundstoffes, welche die erforderliche Kraft bei Dehnung bzw. die elastische Rückstellkraft bestimmt, wird durch die Eigenschaften der Trägerfolie vorgegeben. Die prozentuale Auslängung des Verbundstoffes bis zum Erreichen der Dehngrenze wird durch die Vorverstreckung der nicht elastischen Faseranteile der Deckschicht festgelegt.

Die Deckschicht besteht aus einem Spinnvlies, dessen Filamente einen Filamentkern aus einem thermoplastischen Elastomer und einen Filamentmantel aus einem nicht elastischen thermoplastischen Polymer aufweisen. Die Einzelfilamente sind hierbei als sogenannte Bikomponentenfaser aufgebaut. Geeignete Polymermischungen für den elastomeren Filamentkern umfassen beispielsweise Polyurethane, SIS-, SBS- oder SEBS-Polymere bzw. Mischungen aus diesen Polymeren, als elastische Komponente. Der nicht elastische Filamentmantel kann beispielsweise aus Polypropylen oder Polyethylen bestehen. Der Spinnvliesstoff wird aus Endlosfilamenten hergestellt, die mittels eines Luftstromes unter Ausnutzung eines Venturieffekt aus Spinndüsen abgezogen und in Whirllage auf einem Spinnband abgelegt werden. Das Faservlies wird verfestigt, wobei die Verfestigung mittels eines Kalanders unter Einfluss von Wärme und Druck, durch Vernadeln, Heißluftverfestigung oder andere, dem Fachmann zur Vliesverfestigung bekannte Verfahren erfolgen kann. Eine nachfolgende Querverdehnung des verfestigten Faservlieses bewirkt eine Kaltverstreckung der nicht elastischen Faseranteile. Da das Faservlies Bikomponentenfasern aufweist, die einen elastischen Kern und einen Mantel aus einem nicht elastischen thermoplastischen Polymer aufweisen, kommt es bei der Querverdehnung des verfestigten Faservlieses zu einer Kaltverstreckung des Mantelpolymers der Bikomponentenfasern. Die Schicht des Mantelpolymers wird in Faserrichtung orientiert, wobei das teilweise bis zur Reißgrenze orientierte Mantelpolymer für einen steilen Anstieg der Reißkraftwerte der Bikomponentenfasern sorgt. Nach der Querdehnung stellt sich das Faservlies unter der Wirkung des elastischen Kerns der Bikomponentenfasern elastisch zurück. Durch die beschriebene Vordehnung wird ein elastisches Faservlies mit definierter Dehngrenze erzeugt, welches sich nach Maßgabe der Vordehnung bis zu einer deutlich wahrnehmbaren Dehngrenze elastisch dehnen lässt. Das durch Vordehnung modifizierte Faservlies wird entspannt und nach seiner elastischen Rückstellung ein- oder beidseitig auf die Träger-folie aufkaschiert, welche in Querrichtung ebenfalls elastisch ist.

Die Deckschicht des erfindungsgemäßen Verbundstoffes weist zweckmäßigerweise ein Flächengewicht zwischen 10 g/m² und 200 g/m² auf. Besonders bevorzugt ist ein Flächengewicht zwischen 10 g/m² und 30 g/m². Die einzelnen Fasern besitzen vorzugsweise einen Durchmesser zwischen 10 µm und 30 µm. Gemäß einer bevorzugten Ausführung der Erfindung beträgt der nicht elastische Faseranteil innerhalb des Fasergemisches bis zu 60 Gew.-%, besonders bevorzugt etwa 20 Gew.-%.

Die elastische Trägerfolie hat vorzugsweise ein Flächengewicht zwischen 5 g/m² und 150 g/m². Sie besteht aus einer mehrschichtigen Coextrusionsfolie, die einen Kern aus einem thermoplastischen Elastomer und einer ein- oder beidseitig angeordneten Haftvermittlerschicht zur Verbesserung der Haftung der angrenzenden Deckschicht aufweist. Die Haftvermittlerschicht besitzt zweckmäßigerweise eine hohe Affinität zu Kaschierklebern. Die Dicke der Coextrusionsfolie beträgt vorzugsweise 50 µm bis 150 µm mit einem Dickenverhältnis bei dreischichtigem Aufbau von 1:10:1 bis 1:30:1. Alle Schichten der Coextrusionsfolie können aus elastischen thermoplastischen Polymeren bestehen. Die Haftvermittlerschicht kann durch Additive modifiziert werden, so dass die Trägerfolie nicht verblockt und eine besonders gute Affinität zu Kaschierklebstoffen aufweist. Als Materialien für die Schichten der Coextrusionsfolie können mineralische Füllstoffe sowie Abmischungen von SIS-, SBS-, SEBS- und PU-Polymeren mit Polyolefinen, wie z. B. Polyethylen, Polypropylen, EVA und EBA eingesetzt werden. Die Haftvermittlerschicht kann ferner aus einem rein polyolefinischen Material bestehen. Im Rahmen der Erfindung liegt es jedoch auch, dass coextrudierte Folien in anderen als den angegebenen Schichtenverhältnissen eingesetzt werden.

Die Trägerfolie und die Deckschicht sind durch einen elastischen Schmelzklebstoff verbunden, wobei der Klebstoffauftrag punktförmig, gitterartig, linienförmig in quer zur Dehnungsrichtung verlaufenden Streifen oder vollflächig erfolgen kann. Die verwendeten Schmelzklebstoffe sind vorzugsweise elastische Thermoplaste auf Basis von SIS-, SBS- oder SEBS-Polymeren bzw. deren Mischungen. Der Schmelzklebstoff wird vorzugsweise mit Flächengewichten zwischen 2 g/m² und 20 g/m² zwischen die elastische Trägerfolie und der Deckschicht appliziert. Wenn der Schmelzklebstoff in Linien aufgetragen wird, kann der Abstand der Klebstofflinien 0,2 mm bis 5 mm, bevorzugt 0,5 mm bis 2 mm, betragen. Die Breite der Klebstoffspur beträgt vorzugsweise 0,3 mm bis 2 mm, besonders bevorzugt 0,5 mm bis 1,5 mm.

Gegenstand der Erfindung ist auch ein Verfahren nach Anspruch 6 zur Herstellung des beschriebenen Verbundstoffes. Bevorzugte Ausgestaltungen des Verfahrens sind in den Ansprüchen beschrieben.

Im Folgenden wird die Erfindung anhand einer lediglich ein Ausführungsbeispiel darstellenden Zeichnung ausführlich erläutert. Es zeigen schematisch:
- **Fig. 1a und 1b**: einen erfindungsgemäßen Verbundstoff im ungedehnten sowie im gedehnten Zustand;
- **Fig. 2a und 2b**: ein Filament, aus dem die Deckschicht des Verbundstoffes gefertigt ist,
- **Fig. 3**: ein Dehnungs-Spannungs-Diagramm des Verbundstoffes.

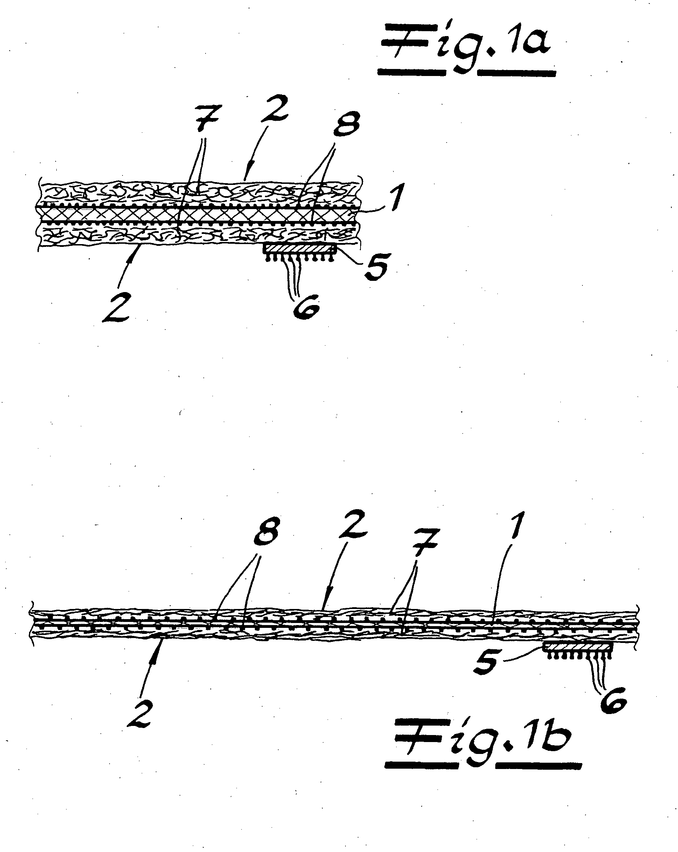

Die Fig. 1a und 1b zeigen einen Verbundstoff für einen elastischen Windelverschluss bestehend aus einer elastischen Trägerfolie 1, die eine bevorzugte Dehnungsrichtung aufweist, und zwei aufkaschierten Deckschichten 2 aus Faservlies, welches Fasern 3 aus einem elastischen Polymer und durch eine Dehnung des Faservlieses 2 in der bevorzugten Dehnungsrichtung verstreckte Faseranteile 4 aus einem nicht elastischen thermoplastischen Polymer enthält.

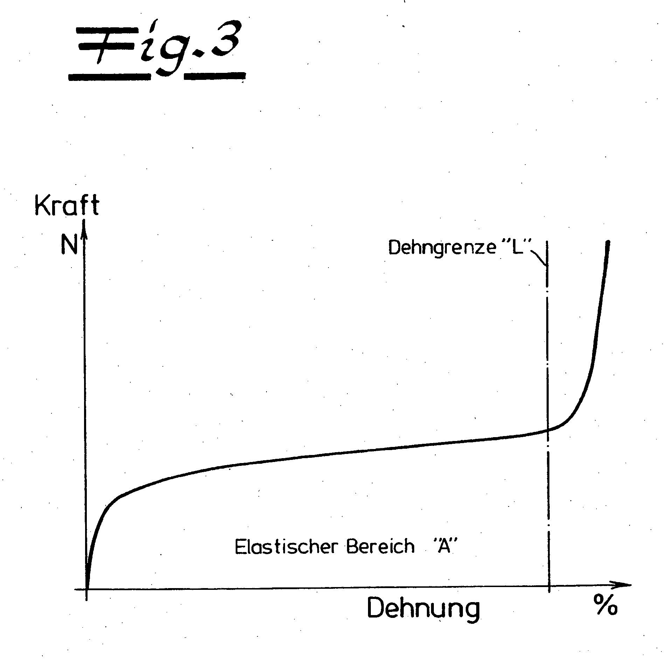

An der Außenseite einer Deckschicht 2 ist ein Hakenband 5 mit männlichen Verschlusselementen 6 befestigt, welches gemeinsam mit weiblichen Verschlusselementen, die im vorderen Bündchenbereich einer nicht dargestellten Windel aufgeklebt sind, einen Klettverschluss bilden. Einer vergleichenden Betrachtung der Fig. 1a und 1b ist zu entnehmen, dass der Verbundstoff in der dargestellten bevorzugten Dehnungsrichtung elastisch dehnbar ist. Der Verbundstoff erlaubt eine elastische Auslängung um 30 % bis 150 % der Ursprungslänge, wobei das Erreichen der elastischen Dehngrenze über einen steilen Kraftanstieg definiert ist. Das Dehnungsverhalten des Materials ist in Fig. 3 dargestellt. In der Fig. 3 ist die zur Dehnung erforderliche Zugkraft des erfindungsgemäßen Verbundstoffes über die Dehnung (Auslängung bezogen auf die Ursprungslänge) aufgetragen. Der Fig. 3 entnimmt man, dass der Verbundstoff bis zur Dehngrenze mit geringer Kraft gedehnt werden kann. Über diesen Verdehnungsbereich stellt sich der Verbundstoff bei Entlastung elastisch zurück. Bei Erreichen der im Material festgelegten Dehngrenze ist ein starker Anstieg der zur weiteren Verdehnung nötigen Kraft festzustellen. Dieser Bereich wird deutlich als nicht elastischer Bereich wahrgenommen.

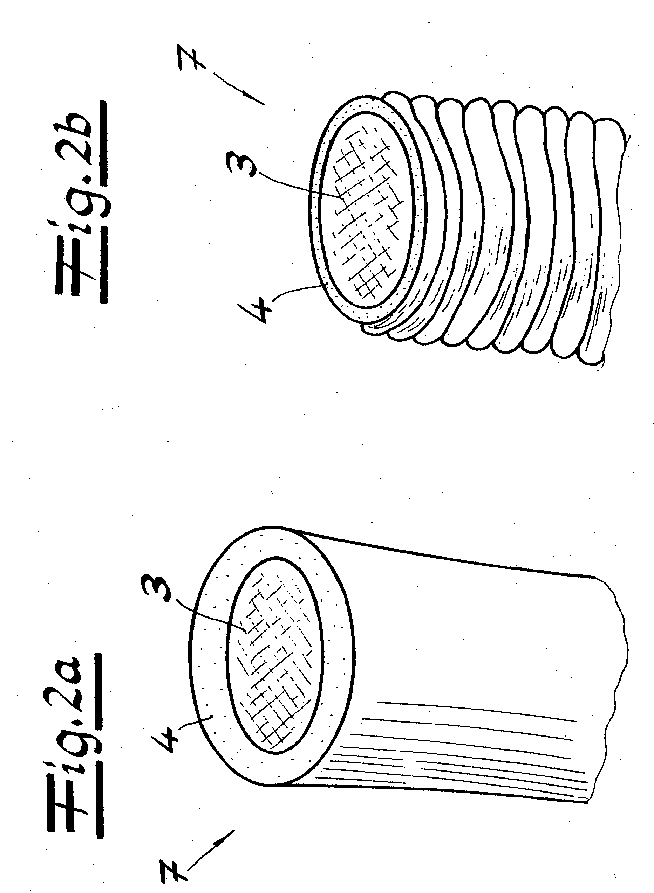

Die Deckschicht besteht aus einem Spinnvlies, dessen Filamente 7 einen Filamentkern 3 aus einem thermoplastischen Elastomer und einem Filament-mantel 4 aus einem nicht elastischen thermoplastischen Polymer aufweisen. Das Faservlies 2 wird durch eine Vorverstreckung modifiziert, bevor es auf die Trägerfolie 1 aufkaschiert wird. Durch die Vordehnung bzw. Vorstreckung ändert sich die Struktur der Filamente. Dies wird aus einer vergleichenden Betrachtung der Fig. 2a und 2b deutlich. Vor der Verstreckung weisen die Filamente 7 des Faservlieses 2 die in Fig. 2a dargestellte Geometrie auf. Durch Vordehnung des Faservlieses wird der Filamentmantel 4 plastisch deformiert, während der Filamentkern 3 lediglich eine elastische Verformung erfährt. Nach der Vordehnung zieht sich das Filament 7 wieder zusammen und besitzt dann die in Fig. 2b dargestellt Kontur. Aufgrund der plastischen Deformation des nicht elastischen Filamentmantels 4 weist dieser nun eine gewellte Struktur auf. Sofern das Filament 7 im Gebrauch gedehnt wird, richtet sich der nicht elastische Filamentmantel 4 zunächst entlang des elastischen Filamentkernes 3 aus und setzt der Dehnung praktisch keinen Widerstand entgegen. Sofern das Filament 7 durch die Dehnung wieder eine ungewellte Kontur erreicht hat, welche im Prinzip der Darstellung in Fig. 2a entspricht, ist die elastische Dehngrenze des Faservlieses 2 erreicht und eine Dehnung über diese Dehngrenze hinaus kann nur erfolgen, wenn der Filamentmantel 4 eine weitere Kaltverstreckung erfährt. Diese zweite Kaltverstreckung ist mit einem steilen Anstieg der zur weiteren Dehnung erforderlichen Kraft verbunden. In diesem Bereich weist das Faservlies 2 daher keine elastischen Eigenschaften mehr auf. Dies wird insbesondere anhand der Fig. 3 deutlich, in der die zur Dehnung erforderliche Zugkraft eines erfindungsgemäßen Verbundstoffes über die Dehnung aufgetragen ist. Im ungedehnten Zustand weisen die Filamente 7 des Verbundstoffes die in Fig. 2b dargestellte Kontur auf. Im elastischen Dehnungsbereich "A" verläuft die Kurve über einen großen Bereich hinweg verhältnismäßig flach, so dass zur Dehnung des Verbundstoffes in diesem Bereich nur vergleichsweise geringe Kräfte erforderlich sind. Beim Erreichen der oberen Grenze des elastischen Bereiches besitzen die Filamente 7 eine Kontur, die sie auch während der maximalen Dehnung bei der Vorverstreckung besaßen. Bei einer Dehnung über die elastische Dehngrenze "L" hinaus steigt die hierzu erforderliche Kraft steil nach oben an, die mit einer weiteren Kaltverstreckung des nicht elastischen Filamentmantels 4 einher geht. Durch die Vorverstreckung der Filamente 7 kann somit die Größe des elastischen Bereiches "A" gezielt eingestellt werden.

Zur Herstellung des erfindungsgemäßen Verbundstoffes wird zunächst eine Bahn aus Faservlies hergestellt, welches elastische Fasern und Faseranteile aus einem nicht elastischen thermoplastischen Elastomer enthält. Dieses wird anschließend in Bahnquerrichtung unter Verstreckung der nicht elastischen Faseranteile gedehnt. Zur Querdehnung wird das Faservlies 2 beispielsweise über einen Reckrahmen oder durch eine Walzenanordnung aus Profilwalzen, z. B. Rillenwalzen, geführt. Hierbei erfolgt eine Dehnung in Bahnquerrichtung um 50 % bis 200 % der ursprünglichen Bahnbreite, wobei die Querdehnung auf die gewünschte Dehngrenze des Verbundstoffes abgestimmt wird. Das gedehnte Faservlies 2 stellt sich nach der Querdehnung elastisch zurück und erreicht weitgehend wieder die Ausgangsbreite.

Das durch Vordehnung modifizierte Faservlies 2 wird ohne Dehnung unter Verwendung eines elastischen Schmelzklebstoffes 8 auf die Trägerfolie 1 aufkaschiert. Der Schmelzklebstoff 8 kann in zur Bahnlängsrichtung parallelen Streifen appliziert werden, wobei die Streifen eine Breite von vorzugsweise 0,3 bis 2 mm, besonders bevorzugt 0,5 bis 1,5 mm, aufweisen und in einem Abstand von vorzugsweise 0,2 bis 5 mm, besonders bevorzugt 0,5 bis 2 mm, auf die elastische Trägerfolie 1 und/oder die Vliesbahn aufgebracht werden.

Der elastische Klebstoff 8 kann SIS-, SBS-, SEBS- oder PU-Polymere bzw. deren Mischungen, enthalten. Zweckmäßigerweise wird der Klebstoff 8 mit einem Auftragsgewicht von 5 bis 10 g/m² auf das Faservlies 2 und/oder die Trägerfolie 1 appliziert. Der Klebstoff 8 kann auch flächig aufgesprüht werden, wobei ein geschlossener Klebstofffilm oder offener, diskontinuierlicher, schuppiger Klebstofffilm erzeugt wird.

Das Faservlies 2 bzw. die Faservliese 2 und die Trägerfolie 1 werden in einem Kaschierwerk mittels zweier Walzen zusammengepresst. Der nach diesem Verfahren hergestellte Verbundstoff ist in Längsrichtung zugfest und verhält sich in Bahnlängsrichtung unelastisch. In Querrichtung verhält sich der Verbundstoff jedoch bis an die durch die erfindungsgemäßen Maßnahmen eingerichtete Dehngrenze nahezu gummielastisch, das heißt der Verbundstoff stellt sich nach der Verdehnung wieder in die ursprüngliche Position zurück. Der so verklebte Verbundstoff wird auf Fertigproduktbreite geschnitten. Für die Verwendung in Windelverschlüssen wird der elastische Verbundstoff mit Hakenbändern 5, selbstklebenden Beschichtungsmassen und nicht haftenden Folien komplettiert und zu Rollen verarbeitet. Der Verbundstoff erlaubt vorzugsweise eine Verdehnung und elastische Rückstellung über einen Gebrauchsbereich von 30 bis 150 %, wobei das Erreichen der elastischen Dehngrenze über den steilen Kraftanstieg definiert ist.

## Patentansprüche

1. Verbundstoff, insbesondere für elastische Windelverschlüsse, bestehend aus einer elastischen Trägerfolie (1) mit einer bevorzugten Dehnungsrichtung und mindestens einer aufkaschierten Deckschicht (2) aus einem Spinnvlies, dessen Filamente (7) einen Filamentkern (3) aus einem thermoplastischen Elastomer und einen Fliamentmantel (4) aus einem nichtelastischen thermoplastischen Polymer aufweisen, **dadurch gekennzeichnet, dass** das Spinnvlies eine definierte Dehngrenze aufweist, die durch Vordehnen in der bevorzugten Dehnungsrichtung erzeugt worden ist und durch eine Verstreckung der nichtelastischen Faseranteile der Deckschicht (2) bestimmt ist, dass die Trägerfolie (1) aus einer mehrschichtigen Koextrusionsfolie besteht, die einen Kern aus einem thermoplastischen Elastomer sowie eine ein- oder beidseitig angeordnete Haftvermittlerschicht zur Verbesserung der Haftung der angrenzenden Deckschicht (2) aufweist, und dass die Trägerfolie (1) mit der Deckschicht (2) durch einen elastischen Schmelzklebstoff (8) verbunden ist.

2. Verbundstoff nach Anspruch 1, **dadurch gekennzeichnet, dass** die Deckschicht (2) ein Flächengewicht zwischen 10 g/m² und 200 g/m² aufweist.

3. Verbundstoff nach Anspruch 1 oder 2, **dadurch gekennzeichnet, dass** der nichtelastische Faseranteil (4) innerhalb des Fasergemisches bis zu 60 Gew.-%, vorzugsweise etwa 20 Gew.-%, beträgt.

4. Verbundstoff nach einem der Ansprüche 1 bis 3, **dadurch gekennzeichnet, dass** die elastische Trägerfolie (1) ein Flächengewicht zwischen 5 g/m² und 150 g/m² aufweist.

5. Verbundstoff nach einem der Ansprüche 1 bis 4, **dadurch gekennzeichnet, dass** der Schmelzklebstoff punktförmig, gitterartig, linienförmig mit in Quer zur Dehnungsrichtung verlaufende Streifen oder vollflächig angeordnet ist.

6. Verfahren zur Herstellung einer Verbundfolie nach einem der Ansprüche 1 bis 5,
wobei aus Filamenten (7), die einen Filamentkern (3) aus einem thermoplastischen Elastomer und einen Filamentmantel (4) aus einem nichtelastischen thermoplastischen Polymer aufweisen, eine Bahn aus einem Faservlies (2) hergestellt wird,
wobei das Faservlies (2) in Bahnquerrichtung unter Verstreckung der nichtelastischen Faseranteile gedehnt wird,
wobei das vorgedehnte Faservlies (2) nach seiner elastischen Rückstellung unter Verwendung eines elastischen Schmelzklebstoffes ein- oder beidseitig auf eine Trägerfolie (1) aufkaschiert wird, die quer zur Bahnlängsrichtung elastisch ist, und
wobei als Trägerfolie (1) eine mehrschichtige Koextrusionsfolie verwendet wird, die einen Kern aus einem thermoplastischen Elastomer und eine ein- oder beidseitig angeordnete Haftvermittlerschicht zur Verbesserung der Haftung des angrenzenden Faservlieses (2) aufweist.

7. Verfahren nach Anspruch 6, **dadurch gekennzeichnet, dass** das Faservlies (2) mechanisch oder thermisch verfestigt und ein Flächengewicht zwischen 10 g/m² und 200 g/m² eingestellt wird.

8. Verfahren nach Anspruch 6 oder 7, **dadurch gekennzeichnet, dass** das Faservlies (2) in Bahnquerrichtung um 50% bis 200% der ursprünglichen Bahnbreite gedehnt wird, wobei die Querdehnung auf die gewünschte Dehngrenze des Verbundstoffes abgestimmt wird.

9. Verfahren nach einem der Ansprüche 6 bis 8, **dadurch gekennzeichnet, dass** das Faservlies (2) zur Querdehnung über einen Reckrahmen wurde durch einen Walzenanordnung aus Profilwalzen geführt wird.

10. Verfahren nach einem der Ansprüche 6 bis 9, **dadurch gekennzeichnet, dass** der Schmelzklebstoff (8) in zur Bahnlängsrichtung parallelen Streifen appliziert wird, wobei die Streifen eine Breite von vorzugsweise 0,3 bis 2 mm aufweisen und in einem Abstand von vorzugsweise 0,2 bis 5 mm auf die elastische Trägerfolie und/oder die Vliesbahn aufgebracht werden.

11. Verfahren nach einem der Ansprüche 6 bis 9, **dadurch gekennzeichnet, dass** der Klebstoff (8) punktförmig auf die zu verbindenden Flächen aufgebracht wird.

12. Verfahren nach einem der Ansprüche 6 bis 9, **dadurch gekennzeichnet, dass** der Klebstoff (8) flächig aufgesprüht wird, wobei ein geschlossener Klebstofffilm oder ein offener, diskontinuierlicher, schuppiger Klebstofffilm erzeugt wird.

## Claims

1. A composite, in particular for elastic nappy closures, consisting of an elastic carrier film (1) with a preferred expansion direction and at least one covering layer (2) laminated thereon and made from a spunbond material, the filaments (7) of which comprise a filament core (3) of a thermoplastic elastomer and a filament mantle (4) of a non-elastic thermoplastic polymer, **characterised in that** the spunbond material has a defined yield strength, which has been created by pre-expansion in the preferred expansion direction and which is determined by a stretching of the non-elastic fibre content of the covering layer (2), **in that** the carrier film (1) consists of a multi-layer co-extrusion film comprising a core of a thermoplastic elastomer as well as an adhesion-promoting layer for improving the adherence of the adjacent covering layer (2), and **in that** the carrier film (1) is connected with the covering layer (2) by an elastic hotmelt adhesive (8).

2. The composite according to claim 1, **characterised in that** the covering layer (2) has an areal weight between 10g/m² and 200/m².

3. The composite according to claim 1 or 2, **characterised in that** the non-elastic fibre content (4) within the fibre mixture is up to 60% by weight, preferably about 20% by weight.

4. The composite according to one of claims 1 to 3, **characterised in that** the elastic carrier film (1) has an areal weight between 5g/m² and 150/m².

5. The composite according to one of claims 1 to 4, **characterised in that** the hotmelt adhesive is arranged in a point-like, grid-like, line-shaped manner with stripes extending transversely to the expansion direction or across the entire surface.

6. A method for manufacturing a composite according to one of claims 1 to 5,
wherein filaments (7) comprising a filament core (3) of a thermoplastic elastomer and a filament mantle (4) of a non-elastic thermoplastic polymer are used to produce a web from a fibre fleece (2),
wherein the fibre fleece (2) is expanded in web transverse direction with stretching of the non-elastic fibre content,
wherein the pre-expanded fibre fleece (2) following its elastic recovery is laminated onto one or both sides of a carrier film (1) which is elastic transversely to the web longitudinal direction, using an elastic hotmelt adhesive, and
wherein the carrier film (1) is a multilayer co-extrusion film which comprises a core of a thermoplastic elastomer and an adhesion-promoting layer arranged on one side or on both sides for improving adhesion of the adjacent fibre fleece (2).

7. The method according to claim 6, **characterised in that** the fibre fleece (2) is mechanically and thermally solidified and an areal weight between 10g/m² and 200/m² is set.

8. The method according to claim 6 or 7, **characterised in that** the fibre fleece (2) is expanded by between 50% and 200% of the original web width in web transverse direction, wherein expansion in transverse direction is adjusted to suit the desired yield strength of the composite.

9. The method according to one of claims 6 to 8, **characterised in that** the fibre fleece (2), for expansion in transverse direction, is guided over a stretching frame or through a roller arrangement consisting of profiled rollers.

10. The method according to one of claims 6 to 9, **characterised in that** the adhesion-promoting material (8) is applied in strips parallel to the web longitudinal direction, wherein the strips have a width of preferably 0.3 to 2mm and are applied to the elastic carrier film and/or the fleece web at a distance of preferably 0.2 to 5mm.

11. The method according to one of claims 6 to 9, **characterised in that** the adhesive (8) is applied to the surfaces to be bonded in a point-like manner.

12. The method according to one of claims 6 to 9, **characterised in that** the adhesive (8) is sprayed on across the entire area, thereby producing a continuous adhesive film or an open, discontinuous, scaly adhesive film.

## Revendications

1. Matière composite, notamment pour des fermetures élastiques de couches-culottes, constituée d'un film support (1) élastique, avec un sens d'extension préférentiel et d'au moins une couche de recouvrement (2) contrecollée en un filé-lié, dont les filaments (7) comportent un noyau de filament (3) en un élastomère thermoplastique et une enveloppe de filament (4) en un polymère thermoplastique non élastique, **caractérisée en ce que** le filé-lié fait preuve d'une limite d'extension définie qui a été créée par une pré-extension dans le sens d'extension préférentiel et qui est définie par un étirage des parts de fibres non élastiques de la couche de recouvrement (2), **en ce que** le film support (1) est constitué d'un film coextrudé multicouches, qui comporte un coeur en un élastomère thermoplastique, ainsi qu'une couche unilatérale ou bilatérale d'agent adhésif pour améliorer l'adhérence de la couche de recouvrement (2) adjacente et **en ce que** le film support (1) est relié avec la couche de recouvrement (2) par une colle fusible (8) élastique.

2. Matière composite selon la revendication 1, **caractérisée en ce que** la couche de recouvrement (2) présente un grammage compris entre 10 g/m² et 200 g/m².

3. Matière composite selon la revendication 1 ou la revendication 2, **caractérisée en ce que** la part en fibres (4) non élastiques à l'intérieur du mélange fibreux s'élève à jusqu'à 60 % en poids, de préférence à environ 20 % en poids.

4. Matière composite selon l'une quelconque des revendications 1 à 3, **caractérisée en ce que** le film support (1) élastique présente un grammage compris entre 5 g/m² et 150 g/m².

5. Matière composite selon l'une quelconque des revendications 1 à 4, **caractérisée en ce que** la colle fusible est placée en forme de points, en grille, en forme de lignes, en rubans s'étendant à la transversale du sens d'extension ou à pleine surface.

6. Procédé de fabrication d'un film composite selon l'une quelconque des revendications 1 à 5,
lors duquel, à partir de filaments (7), qui comportent un noyau de filament (3) en un élastomère thermoplastique et une enveloppe de filament (4) en un polymère thermoplastique non élastique on fabrique une bande en un non-tissé (2),
lors duquel on étend le non-tissé (2) dans la direction transversale de la bande en tirant les parts non élastiques des fibres,
lors duquel, après son retour élastique, on contrecolle le non-tissé (2) pré-étendu en utilisant une colle thermofusible élastique sur une ou sur deux faces d'un film support (1) qui est élastique à la transversale de la direction longitudinale de la bande et
lors duquel on utilise en tant que film support (1) un film coextrudé multicouches qui comporte un coeur en un élastomère thermoplastique et une couche d'agent adhésif disposée sur une ou sur deux faces pour améliorer l'adhérence du non-tissé (2) adjacent.

7. Procédé selon la revendication 6, **caractérisé en ce qu'**on renforce le non-tissé (2) par procédé mécanique ou thermique et **en ce qu'**on règle un grammage compris entre 10 g/m² et 200 g/m².

8. Procédé selon la revendication 6 ou la revendication 7, **caractérisé en ce qu'**on étend le non-tissé (2) dans la direction transversale de la bande de 50% à 200% de la largeur initiale de la bande, l'extension transversale étant adaptée à la limite d'extension souhaitée de la matière composite.

9. Procédé selon l'une quelconque des revendications 6 à 8, **caractérisé en ce que** pour l'extension transversale, on fait passer le non-tissé (2) pardessus un cadre d'extension par un agencement de cylindres à profiler.

10. Procédé selon l'une quelconque des revendications 6 à 9, **caractérisé en ce qu'**on applique la colle fusible (8) en rubans parallèles au sens longitudinal de la bande, les rubans présentant une largeur de 0,3 à 2 mm de préférence et à un écart de 0,2 à 5 mm de préférence, sur le film support élastique et/ou sur la bande de non-tissé.

11. Procédé selon l'une quelconque des revendications 6 à 9, **caractérisé en ce qu'**on applique la colle (8) par points sur les surfaces à relier.

12. Procédé selon l'une quelconque des revendications 6 à 9, **caractérisé en ce qu'**on vaporise la colle (8) à pleine surface en créant un film de colle fermé ou un film de colle ouvert, discontinu, en lamelles.
